Europäisches Patentamt

European Patent Office

Office européen des brevets

(19)

(11) Veröffentlichungsnummer: **0 378 822**

**A1**

(12) **EUROPÄISCHE PATENTANMELDUNG**

(21) Anmeldenummer: **89123312.4**

(22) Anmeldetag: **16.12.89**

(51) Int. Cl.5: **A61F 2/10**

(30) Priorität: **19.01.89 DE 3901435**

(43) Veröffentlichungstag der Anmeldung:
**25.07.90 Patentblatt 90/30**

(84) Benannte Vertragsstaaten:
**BE DE FR NL**

(71) Anmelder: **FIRMA KURT A. MORCHER**
**Kapuzinerweg 12**
**D-7000 Stuttgart 50(DE)**

(72) Erfinder: **Elze, Karl-Ludwig, Dr. med.**
**Hagendornstrasse 47**
**D-2000 Hamburg 13(DE)**

(74) Vertreter: **Manitz, Gerhart, Dr. Dipl.-Phys. et al**
**MANITZ, FINSTERWALD & ROTERMUND**
**Seelbergstrasse 23/25**
**D-7000 Stuttgart 50(DE)**

(54) **Intraoculare Linse.**

(57) Die intraoculare Linse (1) besitzt eine konkave Vorderseite, so daß sowohl bei Fixation der Linse (1) im Sulcus ciliaris (7) als auch im Kapselsack (6) ein besonders großer Abstand zur Iris (5) gegeben ist. Die konvexe Rückseite der Linse (1) schmiegt sich weich an den von ihr ausgespannten Kapselsack (6) an, ohne den Kapselsack (6) zu knicken.

Fig. 3

EP 0 378 822 A1

## Intraoculare Linse

Die Erfindung betrifft eine intraoculare Linse zur Fixation hinter der Iris im Sulcus ciliaris bzw. im eröffneten Kapselsack.

Intraoculare Linsen werden in großem Umfange zur Therapie von grauem Star angewendet. Dabei werden die getrübte Linse des Patienten unter Belassung des Kapselsackes (Linsenkapsel) entfernt und danach die intraoculare Linse eingesetzt.

Grundsätzlich bestehen verschiedene Möglichkeiten, eine intraoculare Linse zu fixieren, wie beispielsweise aus der EP-A 01 36 807 hervorgeht. So kann die intraoculare Linse grundsätzlich vor oder hinter der Iris angeordnet werden, wobei gegebenenfalls eine Befestigung an der Iris durch henkelartige Elemente möglich ist, welche den Öffnungsrand der Iris umgreifen.

In der Regel wird jedoch eine Fixation im Kapselsack oder am Sulcus ciliaris bevorzugt, um Irritationen der Iris nach Möglichkeit zu vermeiden. Für eine derartige Fixation kann der Rand der intraocularen Linse gemäß der EP-A 01 36 807 nach Art eines Flansches ausgebildet sein, dessen Außenrand vom Sulcus ciliaris bzw. vom Kapselsack umspannt wird.

Dabei ist es auch möglich, die von der Iris abgewandte Rückseite der Linse sowie des Flansches mit einer gewissen konvexen Wölbung auszubilden, derart, daß der hinter der intraocularen Linse verbliebene Kapselsack großflächig abgestützt wird.

Statt durch einen Flansch können die intraocularen Linsen im Falle einer Fixation im Kapselsack bzw. im Sulcus ciliaris gemäß dem DE-U 83 36 372 auch mittels elastischer Bügel gehaltert werden, die in Draufsicht auf die Linse etwa C-Form aufweisen, wobei das Krümmungszentrum der C-Bügel etwa in der Ebene der intraocularen Linse im Bereich des Linsenrandes liegt bzw. nach Fixation der Linse im Auge aufgrund der elastischen Verformung der C-Bügel auch nahe der Linsenmitte liegen kann.

Außer in weiter unten dargelegten Sonderfällen müssen intraoculare Linsen die sie durchsetzenden Lichtstrahlen bündeln. Um nun eine möglichst gute Fokussierung der Lichtbündel auf der Netzhaut zu gewährleisten, besitzen herkömmliche intraoculare Linsen eine praktisch ebene oder nur wenig konvex gewölbte, der Netzhaut zugewandte Rückseite und eine konvexe, der Hornhaut zugewandte Vorderseite, deren Krümmungsradius entsprechend der jeweils benötigten Brechkraft der intraocularen Linse bemessen ist.

Falls intraoculare Linsen mit sehr großer Brechkraft benötigt werden, beispielsweise wenn der Augapfel eine ungewöhnlich kurze Länge in Richtung der optischen Achse des Auges besitzt, werden intraoculare Linsen mit besonders starker Krümmung der Vorderseite benötigt. Dies führt dazu, daß die intraoculare Linse im mittleren Bereich eine besonders große Dicke haben muß. Damit besteht aber die Gefahr, daß der Zentralbereich der intraocularen Linse in den Bereich der Iris hineinragt und dieselbe unter Umständen berühren kann. Dies kann zu Irritationen bzw. Schädigungen der Iris führen.

Um dies zu vermeiden, besitzen bisherige Linsen mit großer Brechkraft einen relativ geringen Durchmesser, um eine vergleichsweise geringe Dicke der Linse zu ermöglichen. Damit können aber bei weiter geöffneter Pupille, etwa bei Dämmerung oder Nacht, Lichtstrahlen auf den Linsenrand auftreffen und breite Lichtreflexbilder auf der Netzhaut erzeugen. Zusätzlich treten zwischen dem Linsenrand und dem Rand der Iris Lichtstrahlen direkt zur Netzhaut hindurch. Dadurch wird die Sehleistung unter Umständen ganz erheblich eingeschränkt. Besonders erschwerend kommt hinzu, daß derartige Störungen vor allem bei Dunkelheit auftreten, weil dann die Pupille stark vergrößert ist. Dies hat zur Folge, daß die auf den Linsenrand auftreffenden bzw. zwischen dem Linsenrand und dem Rand der Iris hindurchtretenden Lichtstrahlen, beispielsweise von Autoscheinwerfern, eine extreme Blendgefahr verursachen.

Aus der US-A 47 69 035 sind intraoculare Linsen bekannt, die in Kombination mit der natürlichen Linse verwendet werden sollen und dazu zwischen der natürlichen Linse und der Iris angeordnet werden. Diese intraocularen Linsen besitzen eine der Vorderseite der natürlichen Linse angepaßte konkave Rückseite sowie eine konvexe oder auch konkave Vorderseite, je nachdem, ob zur Korrektur der natürlichen Linse eine intraoculare Linse mit Bündelwirkung oder Streuwirkung benötigt wird. Aufgrund ihrer Anordnung vor der natürlichen Linse besteht zwischen der Vorderseite der intraocularen Linse und der Iris nur ein sehr geringer Abstand, so daß grundsätzlich eine Berührung der intraocularen Linse durch die Iris relativ leicht möglich ist. Dies gilt insbesondere dann, wenn die intraoculare Linse bikonkav ausgebildet ist, weil in diesem Falle der Linsenrand besonders dick sein muß. Die zusätzlich zur natürlichen Linse im Auge eingesetzten intraocularen Linsen sind also wegen möglicher Berührungen mit der Iris nicht unbedenklich.

In der EP-B 00 92 552 werden intraoculare Linsen beschrieben, die wiederum nach Entfernung der natürlichen Linse im Kapselsack oder im Sulcus ciliaris fixiert werden. Diese intraocularen Linsen sind bikonkav ausgebildet und sollen zusammen mit einem Linsensystem außerhalb des Auges (Brillen oder Kontaktschalen) benutzt werden, wobei dieses letztere Linsensystem lichtbündelnd ausgebildet ist. Auf diese Weise wird insgesamt, d.h. durch die Linsen außerhalb und innerhalb des Auges, ein Fernrohrsystem geschaffen, welches dazu dienen soll, degenerative Defekte der Netzhaut auszugleichen. Wenn nämlich der Zentralbereich der Netzhaut, welcher normalerweise feine Strukturen optisch aufzulösen vermag, geschädigt ist, so kann unter Umständen eine hinreichende Sehkraft des Patienten noch dadurch erreicht werden, daß das auf die Netzhaut projizierte Bild stark vergrößert wird. Dies erfolgt nun durch das Zusammenwirken der intraocularen Linse mit externen Linsen. Auf diese Weise läßt sich erreichen, daß der Patient übliche normale Brillen oder Kontaktschalen verwenden kann und nicht auf unschöne Fernrohrbrillen angewiesen ist. Da die intraocularen Linsen gemäß der EP-B 00 92 552 eine Brechkraft von etwa -10 bis -100 Dioptrien aufweisen müssen, besitzen die Linsenränder eine im Vergleich zum Linsendurchmesser große Dicke. Um Berührungen zwischen der intraocularen Linse und der Iris möglichst weitgehend ausschalten zu können, müssen daher oftmals Linsen mit relativ kleinen Durchmessern verwendet werden, mit der Folge, daß bei stärker geöffneter Pupille Lichtreflexionen am Linsenrand bzw. der Durchtritt von Lichtstrahlen zwischen Linsenrand und Öffnungsrand der Iris nicht immer vermeidbar sind.

Außerdem ist nachteilig, daß die intraocularen Linsen der EP-B 00 92 552 bei Anordnung unmittelbar vor dem Kapselsack zu ungewünschten Knickungen des Kapselsackes führen können, weil sich der Kapselsack nur auf dem dicken Linsenrand aufzulegen vermag. Dadurch können Komplikationen auftreten.

Aufgabe der Erfindung ist es nun, eine für die Fixation im Sulcus ciliaris bzw. im Kapselsack geeignete intraocular Linse zu schaffen, mit der Schäden an der Iris unter allen Umständen vermieden und auch bei weit geöffneter Pupille ein gutes Sehvermögen gewährleistet werden können, wobei gleichzeitig eine gute Stabilisierung des Kapselsakkes sichergestellt sein soll.

Diese Aufgabe wird erfindungsgemäß dadurch gelöst, daß die der Iris zugewandte Vorderseite der Linse konkav und die von der Iris abgewandte Rückseite der Linse konvex ist, derart, daß zwischen Iris und Linse - nach Anordnung der Linse im Auge - ein eine unmittelbare Berührung zwischen Linse und Iris sicher ausschließender Abstandsraum verbleibt und der Kapselsack unter Ausspannung von einem Mittelbereich der Linsenrückseite ohne Knickung am Linsenrand abgestützt wird.

Die Erfindung beruht auf der Erkenntnis, daß für eine gute Sehleistung des Auges eine extrem exakte Fokussierung der Lichtstrahlen auf der Netzhaut nur im Netzhautzentrum erforderlich ist, in der Peripherie der Netzhaut ist die Fokussierung vergleichsweise unbedeutend. Die natürliche Linse des Auges hat in dieser Hinsicht auch nur ein begrenztes Leistungsvermögen. Indem bei der Erfindung diese natürlichen Gegebenheiten beachtet werden und auf ein scharf fokussiertes Abbild im Bereich der Netzhautperipherie verzichtet wird, können ganz erhebliche Vorteile erreicht werden.

Zunächst verbleibt aufgrund der konkaven Form der Vorderseite der Linse zwischen Iris und Linse immer ein besonders großer Abstandsraum, welcher eine unmittelbare Berührung zwischen Linse und Iris ausschließt.

Die konkave Vorderseite der Linse bedingt eine ausgeprägt konvexe Form der Linsenrückseite, um die notwendige Bündelwirkung der Linse zu erreichen. Dadurch wird der Vorteil erzielt, daß sich der Kapselsack, welcher auf der Rückseite einer im Sulcus ciliaris bzw. im Kapselsack fixierten intraocularen Linse aufliegt, ohne jede punktuelle Belastung an die Linsenrückseite anschmiegen kann und gleichmäßig ausgespannt wird. Durch entsprechende Bemessung der Wölbungen von Vorder- und Rückseite der Linse kann dabei gleichzeitig gewährleistet werden, daß keinerlei Knickung des Kapselsackes am Linsenrand aufzutreten vermag. Es läßt sich nämlich erreichen, daß die Berührungsfläche zwischen Linsenrückseite und Kapselsack vom Äquator der intraocularen Linse durch eine ringförmige Abstandszone beabstandet ist, in deren Bereich der Kapselsack mit geringer Entfernung von der Linse erstreckt ist.

Außerdem ist vorteilhaft, daß die gewölbeartige Form der intraocularen Linse, bei der die konvexe Gewölbeseite dem Augeninneren zugewandt ist, die Lage des im Auge befindlichen Glaskörpers stabilisiert, und zwar in ähnlicher Weise, wie es die natürliche Linse vor der Operation bewirkt.

Somit spannt die erfindungsgemäße Linse einerseits die hintere Linsenkapsel gleichmäßig aus und verlagert andererseits den konstruktionsbedingten Linsenschwerpunkt vergleichsweise weit nach hinten in Richtung des Augeninneren. Damit wird verhindert, daß die hintere Linsenkapsel nach der Linsenkernentfernung locker wie ein Vorhang verbleibt und der Glaskörper eine größere Bewegungsfreiheit hat, was zu Komplikationen führen könnte.

Ein weiterer Vorzug der Erfindung besteht darin, daß die intraoculare Linse auch bei besonders hoher Brechkraft einen großen Durchmesser aufweisen kann. Die bei großen Brechstärken notwendige ausgeprägte konvexe Wölbung der Linsenrückseite ist besonders erwünscht, da sie den na-

türlichen Krümmungsverlauf der hinteren Linsen-kapsel imitiert, denn bei der natürlichen menschlichen Linse ist die Rückseite stärker gewölbt als die Vorderseite.

Im übrigen erleichtert die erfindungsgemäße Linse das Sehen bei Dunkelheit. Da sowohl die Vorder- als auch die Rückseite der Linse gewölbt und von der Linsenfrontseite aus gesehen konkav sind, können bei Gegenlicht, beispielsweise durch Scheinwerfer von Autos, allenfalls punktuelle Reflexe an den Linsen auftreten, so daß Blendungserscheinungen deutlich vermindert werden.

Die erfindungsgemäße Form der intraocularen Linsen ist für verschiedene Arten der Fixation gleichermaßen geeignet. Beispielsweise können die intraocularen Linsen einen derart großen Durchmesser haben, daß sie ohne gesonderte Fixierorgane hinreichend fest in den Kapselsack des Auges eingesetzt werden können.

Andererseits ist es auch möglich, an der intraocularen Linse eine Haptik anzuordnen, beispielsweise bogen- bzw. C-förmige Bügel, die die Linse am Äquator des Kapselsackes bzw. im Sulcus ciliaris abstützen und verankern.

Vorzugsweise sind die Haptikelemente gegenüber einer den Linsenrand enthaltenden Ebene der intraocularen Linse zur Linsenvorderseite hin abgewinkelt, so daß die Haptikelemente die gewölbeartige Form der Linse fortsetzen und der Linsenschwerpunkt besonders weit nach hinten, zum Augeninneren hin, verlagert wird.

Im übrigen wird hinsichtlich bevorzugter Merkmale der Erfindung auf die Ansprüche sowie die nachfolgende Beschreibung einer besonders bevorzugten Ausführungsform verwiesen, die in der Zeichnung dargestellt ist. Dabei zeigt

Fig. 1 eine Draufsicht auf die erfindungsgemäße intraoculare Linse,

Fig. 2 eine Seitenansicht derselben und

Fig. 3 ein ausschnittsweises Schnittbild eines Auges mit darin im Sulcus ciliaris fixierter intraocularer Linse.

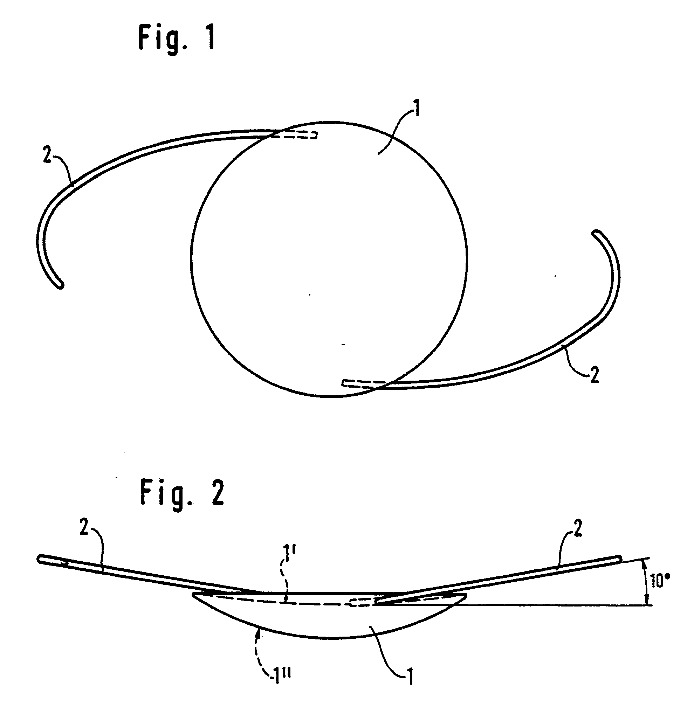

Die erfindungsgemäße intraoculare Linse 1 besitzt gemäß den Figuren 1 und 2 eine konkave Vorderseite 1′ und eine konvexe Rückseite 1″. Zur Halterung der Linse im Auge sind am Linsenrand als Haptik C-förmige Bügel 2 angeordnet, deren Krümmungszentrum linsenseitig liegt und die gegenüber einer den Linsenrand enthaltenden Ebene um ca. 10° in Richtung der Linsenvorderseite abgewinkelt sind.

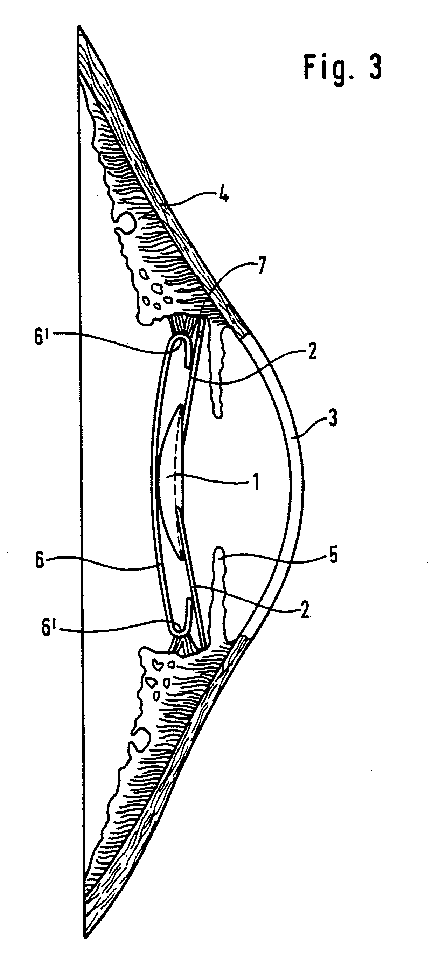

In Fig. 3 ist vom menschlichen Auge lediglich der an die Hornhaut 3 anschließende Abschnitt dargestellt. Hinter der transparenten Hornhaut, welche ringförmig von der Lederhaut 4 umgeben wird, befindet sich die Iris 5, dahinter der Kapselsack 6. Die der Iris 5 zugewandte Vorderseite des Kapselsackes 6 ist ebenso wie die bei gesundem Auge vom Kapselsack umschlossene natürliche Linse des Auges entfernt. Stattdessen ist die intraoculare Linse 1 angeordnet, wobei die Bügel 2 im Raum zwischen der Iris 5 und dem Kapselsack 6 im Sulcus ciliaris 7 gehalten sind.

Stattdessen ist es auch möglich, die Bügel 2 in den Kapselsack 6 einzusetzen, derart, daß sie sich etwa am Äquator 6′ des Kapselsackes 6 abstützen.

Aufgrund der konkaven Vorderseite der Linse 1 wird ein besonders großer Abstand zwischen Iris 5 und Linse 1 gewährleistet. In dieser Hinsicht ist auch die Abwinklung der Bügel 2 zur Linsenvorderseite hin vorteilhaft. Die konvexe Rückseite der Linse 1 bildet eine kuppenförmige Schmiegfläche für den Kapselsack 6, so daß derselbe von der Linse 1 einerseits unter leichter Spannung gehalten werden kann, andererseits aber an keiner Stelle, etwa am Linsenrand, scharfkantig geknickt werden kann.

Der relativ große Abstand der Linse 1 zur Iris 5 sowie die gute Abstützung des Kapselsackes 6 auf der Linsenrückseite bleiben auch dann gewährleistet, wenn die Linse 1 einen besonders großen Durchmesser und/oder eine besonders stark gewölbte Rückseite und damit eine besonders große Dicke aufweist.

Abweichend von der dargestellten Ausführungsform kann die Linse 1 auch derart groß ausgeführt sein, daß sie den Kapselsack 6 praktisch völlig ausfüllt und mit ihrem entsprechend abgerundeten Rand unmittelbar, ohne zusätzliche Halteelemente, am Äquator 6′ des Kapselsackes 6 fest gehalten wird.

Die Linse 1 sowie die Bügel 2 können aus Polymethylmethacrylat bestehen.

## Ansprüche

1. Intraoculare Linse (1) zur Fixation hinter der Iris (5) im Sulcus ciliaris (7) bzw. im eröffneten Kapselsack (6), dadurch gekennzeichnet, daß die der Iris (5) zugewandte Vorderseite (1′) der Linse (1) konkav und die von der Iris (5) abgewandte Rückseite (1″) der Linse (1) konvex ist. derart, daß zwischen Iris (5) und Linse (1) - nach Anordnung der Linse (1) im Auge - ein eine unmittelbare Berührung zwischen Linse (1) und Iris (5) sicher ausschließender Abstandsraum verbleibt und der Kapselsack (6) unter Ausspannung von einem Mittelbereich der Linsenrückseite (1″) ohne Knickung am Linsenrand abgestützt wird.

2. Linse nach Anspruch 1, dadurch gekennzeichnet, daß am Linsenrand eine Haptik zur Fixation angeordnet ist, z.B. in Form C-förmiger Bügel (2) mit linsenseitigem Krümmungszentrum.

3. Linse nach einem der Ansprüche 1 oder 2, gekennzeichnet durch einen Durchmesser von mehr als 6,5 mm.

4. Linse nach einem der Ansprüche 1 bis 3, gekennzeichnet durch einen Durchmesser von mehr als 7 mm.

5. Linse nach einem der Ansprüche 2 bis 4, dadurch gekennzeichnet, daß die Elemente (2) der Haptik gegenüber einer den Linsenrand enthaltenden Ebene der intraocularen Linse (1) um etwa 10° zur Linsenvorderseite hin abgewinkelt sind.

EP 0 378 822 A1

# Fig. 1

# Fig. 2

EP 0 378 822 A1

Fig. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 738 680 (W.K. HERMAN)<br>* Spalte 2, Zeilen 29-43; Spalte 4, Zeilen 48-63; Spalte 5, Zeilen 59-62; Abbildungen 1,2A,3 * | 1,2 | A 61 F 2/16 |
| Y | | 3-5 | |
| Y | US-A-4 718 904 (S.P. THORNTON)<br>* Zusammenfassung; Spalte 3, Zeilen 8-16; Abbildungen *<br>--- | 3 | |
| Y | WO-A-8 504 566 (LENS S.R.L. et al.)<br>* Seite 9, Zeilen 7-19; Abbildung 5 *<br>--- | 4 | |
| Y | WO-A-8 301 568 (SURGIDEV)<br>* Seite 8, Zeilen 31-33; Seite 10, Zeilen 14-21; Abbildungen 1,2,4,5 * | 5 | |

**RECHERCHIERTE SACHGEBIETE (Int. Cl.5)**

A 61 F

Der vorliegende Recherchenbericht wurde für alle Patentansprüche erstellt

| Recherchenort | Abschlußdatum der Recherche | Prüfer |
|---|---|---|
| DEN HAAG | 09-02-1990 | WOLF C.H.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
............................................................
& : Mitglied der gleichen Patentfamilie, übereinstimmendes Dokument

EPO FORM 1503 03.82 (P0403)